(19) Europäisches Patentamt
European Patent Office
Office européen des brevets

(11) Veröffentlichungsnummer: **0 248 361 B1**

(12) # EUROPÄISCHE PATENTSCHRIFT

(45) Veröffentlichungstag der Patentschrift: **08.01.92**

(51) Int. Cl.[5]: **A61K 31/545**, //(A61K31/545, 31:43)

(21) Anmeldenummer: **87107803.6**

(22) Anmeldetag: **29.05.87**

Die Akte enthält technische Angaben, die nach dem Eingang der Anmeldung eingereicht wurden und die nicht in dieser Patentschrift enthalten sind.

(54) **Pharmazeutische Zubereitung zur Behandlung bakterieller Infektionen.**

(30) Priorität: **04.06.86 DE 3618813**

(43) Veröffentlichungstag der Anmeldung:
**09.12.87 Patentblatt 87/50**

(45) Bekanntmachung des Hinweises auf die Patenterteilung:
**08.01.92 Patentblatt 92/02**

(84) Benannte Vertragsstaaten:
**AT BE CH DE ES FR GB GR IT LI LU NL SE**

(56) Entgegenhaltungen:
**EP-A- 120 613**
**GB-A- 2 017 493**

**BIOLOGICAL ABSTRACTS, Band 72, 1981, Seite 217, Nr. 223, Biological Abstracts Inc., Philadelphia, US; A. IMADA et al.: "Taxonomy of the producing strain, fermentation and antibacterial properties", & J. ANTIBIOT (TOKYO) 33(12): 1417-1424 1980**

(73) Patentinhaber: **HOECHST AKTIENGESELLSCHAFT**
**Postfach 80 03 20**
**W-6230 Frankfurt am Main 80(DE)**

(72) Erfinder: **Limbert, Michael, Dr.**
**Am Alten Birnbaum 21**
**W-6238 Hofheim am Taunus(DE)**
Erfinder: **Schrinner, Elmar, Dr.**
**Hedwigstrasse 2**
**W-6200 Wiesbaden(DE)**
Erfinder: **Seibert, Gerhard, Prof. Dr.**
**Gläserweg 21**
**W-6100 Darmstadt(DE)**

**EP 0 248 361 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß sich Antibiotoka aus der Klasse der Cephalosporine, wie z.B. Cefotaxim, Cefodizim oder Cefpirom hervorragend zur Therapie bakterieller Infektionen eignen, daß jedoch die antibakterielle Aktivität gegenüber bestimmten grampositiven und gramnegativen aeroben, insbesondere anaeroben Keimen nicht ausreichend sein kann.

Antibiotika aus der Gruppe der Peneme, wie z.B. die aus der EP-A 0170028 bekannte 5R,6S-6-(1R-Hydroxyethyl)-3-(4-carbamoyl-phenoxy)-7-oxo-4-thia-1-azabicyclo[3.2.0]hept-2-en-carbonsäure haben eine antibakterielle Aktivität, die viele Krankheitserreger abtötet, gegen die Cephalospcrine nur wenig wirksam sind.

Aus der EP-A-0 120 613 ist bekannt, daß zwischen bestimmten Penemen und Penicillinen oder Cephalosporinen synergistische Wirkungen auftreten können. Gegenüber den dort beschriebenen Penem-Cephalosporin-Kombinationen zeichnen sich die erfindungsgemäßen Zubereitungen durch eine wesentlich höhere antibakterielle Aktivität aus.

In J. Antiobiot. 33 (1980), Nr. 12, S. 1417-1424, wird beschrieben, daß die Carbapeneme C 19393 $S_2$ und $H_2$ $\beta$-Lactamaseinhibitoren darstellen, die mit Ampicillinen und Cefotiam synergistisch wirksam sind gegen klinische Isolate, die eine Resistenz gegen $\beta$-Lactamantibiotika aufweisen.

GB-A-2 017 493 ist zu entnehmen, daß Cefoperazon und bestimmte Derivate zusammen mit $\beta$-Lactamaseinhibitoren, die einen $\beta$-Lactamring haben, wie ganz bestimmte Penicilline, Cephalosporine oder Clavulansäure, eine synergistische Wirkung zeigen.

Es wurde nun gefunden, daß Cephalosporine in Kombination mit bestimmten Penemen überraschenderweise einen deutlich synergistischen antibakteriellen Effekt haben.

Die Erfindung betrifft daher pharmazeutische Zubereitungen mit synergistischer antibakterieller Wirksamkeit, enthaltend ein Cephalosporin und ein Penemantibiotikum, die dadurch gekennzeichnet sind, daß

(a) ein Cephalosporinderivat der allgemeinen Formel I

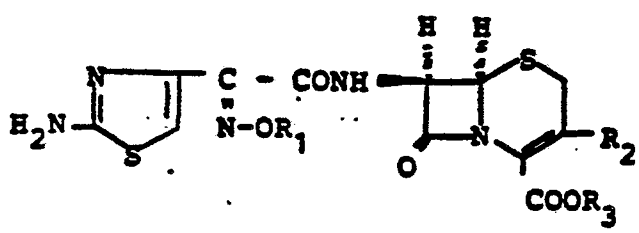

(I) .

worin

$R_1$ für Wasserstoff, $C_1$-$C_4$-Alkyl oder Carboxy-$C_1$-$C_4$-alkyl steht und die Gruppe $=N-OR_1$ in syn-Position steht,

$R_2$ die Bedeutung hat von Wasserstoff, Methyl, Methoxy, Vinyl, Acetoxymethyl, Carbamoyloxymethyl, von $-CH_2S-X$,
mit X =

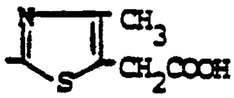 , 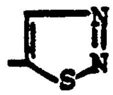 , 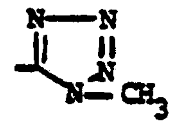 , 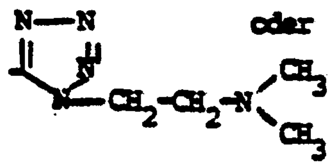 oder 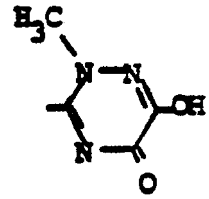

von 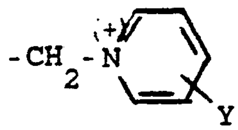 mit Y = Wasserstoff, $C_1$-$C_4$-Alkylthio, $C_1$-$C_4$-Alkoxy oder $C_3$-$C_5$-Cycloalkyl,

von 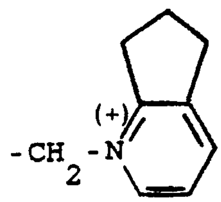 oder 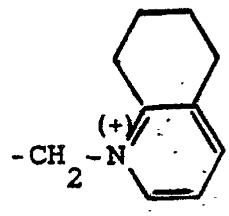 ,

wobei die ankondensierten Ringe auch in 3,4-Stellung stehen und auch durch Sauerstoff unterbrochen sein können,
von

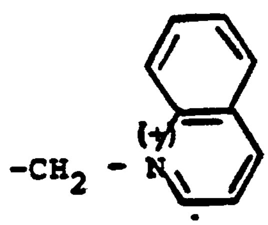 oder 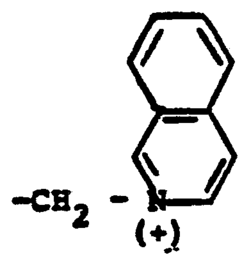

von Thienopyridinio-methyl, Furopyridinio-methyl oder von 5-Methyl-tetrazol-2-yl-methyl
und
$R_3$ für Wasserstoff, ein physiologisch verträgliches Kation, einen physiologisch verträglichen Esterrest oder - falls in $R_2$ die Struktur

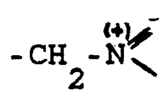

auftritt - für eine negative Ladung steht, und
(b) ein Penem-Antibiotikum der allgemeinen Formel II

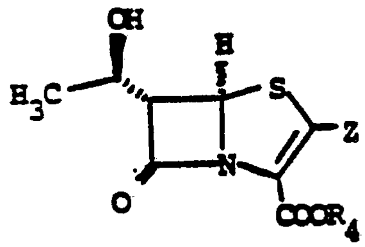

(II)

worin

Z steht für 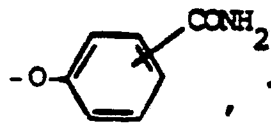, 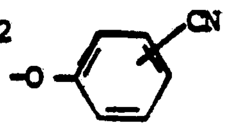, 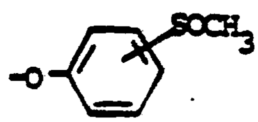,

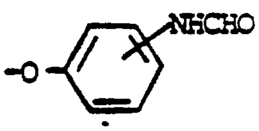, 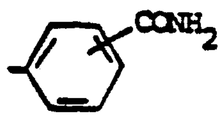, 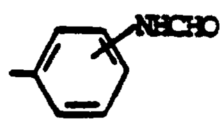, 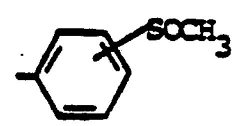,

$-CH_2-O-CONH_2$, $-S-(CH_2)_2-O-CONH_2$ oder $-S-(CH_2)_3-SO_2NH_2$ und

$R_4$ für Wasserstoff, ein physiologisch verträgliches Kation oder einen physiologisch verträglichen Esterrest,

vorliegen.

Erfindungsgemäß eingesetzte Aminothiazol-Cephalosporine und deren physiologisch verträgliche Salze oder Ester werden beispielsweise beschrieben in den Deutschen Offenlegungsschriften Nr. 27 02 501, 27 13 272, 27 15 385, 28 10 922, 29 21 316, 29 22 036, in EP-A 0064740, in U.S. 4 278 793, U.S. 4 501 739, in GB-A 2 105 334 und GB-A 2 105 335.

Penem-Antibiotika der Formel II werden beispielsweise beschrieben in EP-A 0069377, EP-A 0170028, GB-A 85.20631, EP-A 121502, GB 2 097 786 und BE 898 603.

Steht $R_1$ in Formel I für $C_1$-$C_4$-Alkyl, so kommen z.B. Methyl, Ethyl, Propyl in Betracht, vorzugsweise Methyl.

Steht $R_1$ für Carboxy-$C_1$-$C_4$-alkyl, so sind z.B. Carboxymethyl, Carboxyethyl, Carboxypropyl, vorzugsweise der Rest $-CH_2-COOH$, insbesondere jedoch der Rest

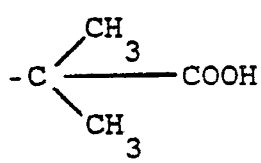

von Interesse.

Von den vorstehend aufgeführten substituierten Pyridiniummethyl-Resten sind bevorzugt 2,3-Cyclopenteno- und 2,3-Cyclohexeno-pyridinium-methyl, sowie 4-Methylthio-, 4-Cyclopropyl- und 3-Methoxy-pyridinium-methyl, sowie ebenfalls 3,4-Cyclopenteno- und 3,4-Cyclohexeno-pyridinium-methyl.

Erfindungsgemäß von ganz besonderem Interesse sind diejenigen Verbindungen der Formel I, in der $R_1$ für Methyl und $R_2$ für $-CH_2-OCOCH_3$ (Cefotaxim),

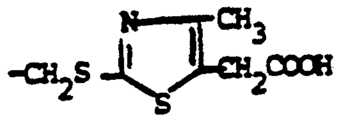 (Cefodizim),

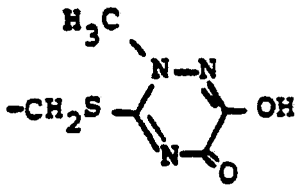 (Ceftriaxon),

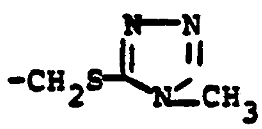 (Cefmenoxim) oder

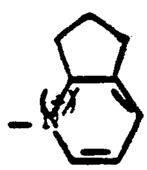 (Cefpirom) steht, oder

$R_1$ für $-CH_2COOH$ und $R_2$ für $-CH=CH_2$ (Cefixim) steht, wobei innerhalb dieser Gruppe das Cefodizim, Cefpirom, Ceftriaxon, insbesondere das Cefotaxim nochmals eine Vorzugsstellung einnimmt.

Steht $R_2$ für eine $-CH_2$-Pyridinium-Verbindung, so liegt in der allgemeinen Formel I die Carboxylgruppe als inneres Salz ($-COO^{(-)}$) vor.

Von den Penem-Antibiotika der Grundstruktur B sind besonders bevorzugt solche der allgemeinen Formel II

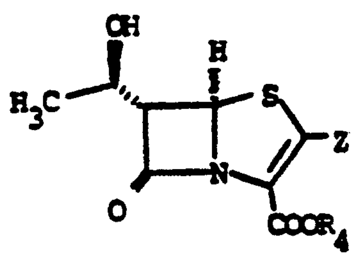 (II)

worin
Z stehen kann für

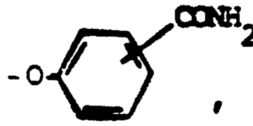, 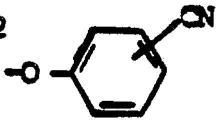, 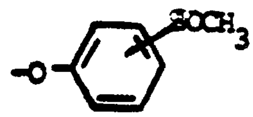,

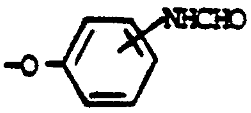, 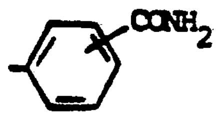, 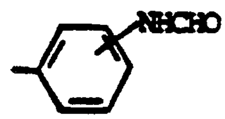, 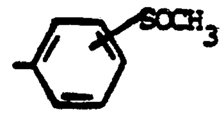,

$-CH_2-O-CONH_2$, $-S-(CH_2)_2-O-CONH_2$ oder $-S-(CH_2)_3-SO_2NH_2$ und $R_4$ für Wasserstoff, ein physiologisch verträgliches Kation oder einen physiologisch verträglichen Esterrest. Von den vorstehend aufgeführten, substituierten Phenoxy- und Phenyl-Substituenten sind bevorzugt die Reste

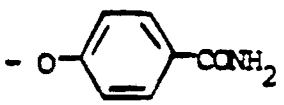, 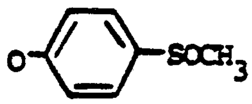, 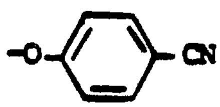, 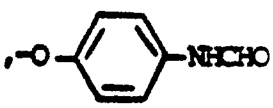

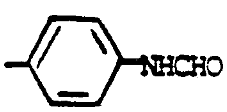, 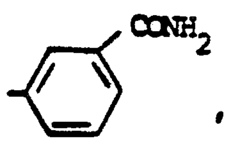, 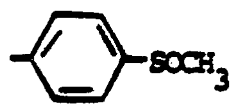 und 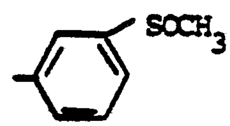

wobei der 4-Carbamoyl-phenoxyrest (HRE 664) noch besonders hervorzuheben ist.

Erfindungsgemäß ganz besonders interessant ist demnach eine Kombination von Cefodizim oder Cefpirom, insbesondere jedoch von Cefotaxim und dem Penem HRE 664.

$R_3$ und $R_4$ können stehen für Wasserstoff oder ein physiologisch verträgliches Kation, wie z.B. ein Alkalikation, vorzugsweise Kalium oder Natrium, insbesondere Natrium, oder auch andere literaturbekannte, physiologisch verträgliche Salze mit Erdalkali- oder organischen Ammoniumionen (vgl. z.B. U.S. 4 278 793).

$R_3$ und $R_4$ können weiterhin stehen für einen insbesondere für die enterale Applikation interessanten, physiologisch verträglichen Esterrest, wie z.B. für einen Acyloxymethyl- oder Acyloxyethylrest mit 2 bis 12, vorzugsweise 2 bis 6 C-Atomen im Acylteil, vorzugsweise Acetoxymethyl, 1'-(Acetoxy)ethyl oder Pivaloyloxymethyl, für 5-Methyl-1,3-dioxalen-2-on-4-yl-methyl, oder auch für andere, physiologisch verträgliche Ester, wie sie beispielsweise in EP-A 0170028 beschrieben sind.

Die Herstellung der erfindungsgemäß in Betracht kommenden Komponenten der Wirkstoffkombination wird beispielsweise in den vorstehend genannten Patentrechten beschrieben.

Die erfindungsgemäße Kombination von Cephalosporinen und Penemen wirkt stark antibakteriell und ist deshalb zur Behandlung bakterieller Infektionen besonders gut geeignet. Wesentlich ist die Tatsache, daß die Wirkung der beiden Komponenten sich nicht additiv verhält, sondern vielmehr ein unerwarteter, starker synergistischer Effekt auftritt.

Selbst bei Keimen, gegen die eine Einzelkomponente allein keine signifikante antibakterielle Wirkung hat, ist in Kombinationen ein synergistischer Effekt zu beobachten.

Die erfindungsgemäßen Zubereitungen erfassen somit ein Keimspektrum bei niedrigen minimalen Hemmkonzentrationen, die von den Einzelkomponenten nicht erreicht werden.

Aus den genannten Gründen ist die erfindungsgemäße Zubereitung den Einzelkomponenten bei der Behandlung bakterieller Infektionen überlegen. Sie erlaubt es, geringere Dosierungen der Einzelkomponenten zuzuführen und dennoch einen besseren Therapieerfolg zu erzielen.

Die Erfindung betrifft auch ein Verfahren zur Herstellung einer solchen Zubereitung, das dadurch gekennzeichnet ist, daß man

a) ein Cephalosporinderivat der allgemeinen Fomel I gemäß Anspruch 1 oder dessen physiologisch verträgliche Salze oder Ester und

b) ein Penem-Antibiotikum der allgemeinen Formel II gemäß Anspruch 1 oder dessen physiologisch verträgliche Salze oder Ester

zusammen mit (einem) physiologisch annehmbaren Träger(n) und gegebenenfalls weiteren Hilfs- oder Zusatzstoffen in eine geeignete Darreichungsform bringt.

Die Erfindung betrifft weiterhin ganz allgemein pharmazeutische Zubereitungen, die nebeneinander, in unvermischter Form

a) ein Cephalosporinderivat der allgemeinen Formel I gemäß Anspruch 1 oder dessen physiologisch verträgliche Salze oder Ester und

b) ein Penem-Antibiotikum der allgemeinen Formel II gemäß Anspruch 1 oder dessen physiologisch verträgliche Salze oder Ester enthalten und

Kombinationspräparate zur gleichzeitigen, getrennten oder zeitlich abgestuften Anwendung bei der Behandlung bakterieller Infektionen darstellen.

Die Dosen des Cephalosporinderivats und des Penem-Antibiotikums werden in den erfindungsgemäßen Zubereitungen vorzugsweise so gewählt, daß die Einzelkomponenten noch keine ausreichende oder volle Wirkung zeigen würden. Die Tagesdosis der erfindungsgemäßen Kombinationen (Summe der Einzelkomponenten) liegt zwischen 1 bis 16 g, vorzugsweise 4 bis 8 g. Das Verhältnis der Einzelkomponenten in der Kombination kann zwischen 1:9 und 9:1, vorzugsweise zwischen 1:5 und 5:1 liegen. Die Dosis in einer Verabreichungseinheit kann beispielsweise zwischen 50 und 2000 mg gewählt werden.

Da die Herstellung der Cephalosporinkomponente im allgemeinen weniger aufwendig und damit auch preiswerter ist als die Herstellung des Penem-antibiotikums wird man schon aus diesem Grunde eine erfindungsgemäße Kombination vorziehen, in der der Cephalosporinanteil höher liegt als der des Penems.

Die erfindungsgemäßen Zubereitungen bzw. Erzeugnisse können parenteral oder oral verabreicht werden. Bevorzugt ist die parenterale Applikation.

Die chemotherapeutisch verwendbaren, erfindungsgemäßen Kombinationen der Wirkstoffe können zur Herstellung von pharmazeutischen Präparaten verwendet werden, welche eine wirksame Menge der Aktivsubstanzen, zusammen mit Trägerstoffen enthalten und die sich zur enteralen und parenteralen Verabreichung eignen. So sind injizierbare Lösungen vorzugsweise isotonische wässrige Lösungen oder Suspensionen, die sterilisiert sein können und Hilfsstoffe, wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere chemotherapeutisch wertvolle Stoffe enthalten können, werden z.B. mittels konventioneller Verfahren hergestellt.

Die erfindungsgemäße Kombination wird zur parenteralen Applikation vorzugsweise unmittelbar vor der Anwendung in sterilem Wasser oder - falls erforderlich - einer Pufferlösung, wie beispielsweise einem Phosphat- oder Carbonatpuffer, wie er üblicherweise für diese Zwecke zum Einsatz kommt, gelöst und anschließend appliziert.

Die folgenden Beispiele dienen zur Erläuterung der Erfindung.

Beispiel 1

Wirkung der Kombination des Natriumsalzes von 5R,6S-6-(1R-Hydroxyethyl)-3-(4-carbamoylphenoxy)-7-oxo-4-thia-1-azabicyclo[3.2.0]hept-2-en-carbonsäure (HRE 664, in der Tabelle als (1) bezeichnet) und des Natriumsalzes von Cefotaxim (2) gegen grampositive und gramnegative Teststämme. Zur Ermittlung des Synergismus wurden Mischungen von der Verbindung (1) mit der Verbindung (2) im Verhältnis 1:1, 1:2 und 1:3 hergestellt. Die MHK-Werte wurden im Vergleich zu den Einzelsubstanzen geprüft.

Tabelle 1

| Keim | MHK in mg/l | | | | |
|---|---|---|---|---|---|
| | (1) alleine | (2) alleine | (1) : (2) | | |
| | | | 1:1 | 1:2 | 1:3 |
| Staph. SG 511 | 0,062 | 2 | 0,125 | 0,125 | 0,125 |
| Staph. 285 | 0,062 | 2 | 0,125 | 0,125 | 0,125 |
| Staph. 503 | 0,062 | 2 | 0,125 | 0,125 | 0,125 |
| Kl.aerog. 1082E | 0,5 | 1 | 0,5 | 0,5 | 0,5 |
| Citrob. 82 Cull. | 1 | 2 | 0,5 | 0,5 | 0,25 |

Beispiel 2

Wirkung der Kombination von (1) und (2) gegen klinische Isolate mit Resistenz gegen die Cephalosporinkomponente.

Tabelle 2

| Keim | MHK in mg/l | | | | |
|---|---|---|---|---|---|
| | (1) alleine | (2) alleine | (1) : (2) | | |
| | | | 1:1 | 1:2 | 1:3 |
| Staph. 789 | 4 | 64 | 4 | 4 | 8 |
| Staph. 22130 | 2 | 32 | 4 | 4 | 4 |
| Strept. D 756 | 8 | 64 | 4 | 4 | 4 |
| Strept. D Eder | 8 | 64 | 4 | 4 | 4 |
| Strept.faecium D | 8 | 64 | 8 | 16 | 8 |
| Citrob. 2901 | 8 | 64 | 4 | 4 | 4 |
| Ent. cl. M 423 | 4 | 64 | 2 | 2 | 4 |
| Ent. cl. M. 447 | 16 | 64 | 16 | 8 | 8 |
| Ent. cl. P99 | 2 | 64 | 2 | 2 | 2 |

Beispiel 3

Wirkung der Kombination von (1) und (2) gegen anaerobe Keime

Tabelle 3

| Keim | MHK in mg/l | | | | |
|---|---|---|---|---|---|
| | (1) alleine | (2) alleine | (1) : (2) | | |
| | | | 1:1 | 1:2 | 1:3 |
| B.fragilis 312 | 0,125 | 128 | 0,125 | 0,25 | 0,25 |
| B.fragilis 960 | 0,125 | 16 | 0,125 | 0,125 | 0,125 |
| B.fragilis 17390 | 0,25 | 8 | 0,25 | 0,5 | 0,25 |
| B.fragilis 18125 | 0,25 | 2 | 0,25 | 0,25 | 0,25 |
| B.ovatus 103 | 0,125 | 64 | 0,125 | 0,125 | 0,125 |
| B.vulgatus 1446 | 0,125 | 64 | 0,125 | 0,125 | 0,125 |
| B.thetaiotaomicron 123 | 0,125 | 64 | 0,125 | 0,125 | 0,125 |
| Sph. varius 5262 | 0,25 | 64 | 0,5 | 1 | 1 |
| Cl. tetani 19406 | 0,25 | 8 | 0,5 | 0,5 | 0,5 |

Beispiel 4

Die Kombination des Kaliumsalzes der Penem-Verbindung 5R,6S-3-(2-Carbamoyloxy-ethylthio)-6-(1R-hydroxyethyl)-7-oxo-4-thia-1-azabicyclo[3.2.0]hept-2-en-carbonsäure (3) mit der Verbindung (2) wurde bei 3 Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte 0,5 für eine synergistische Aktivität charakteristisch, FIC-Werte von 1 zeigen eine additive Interaktion.

Tabelle 4

| Stamm | FIC-Werte einer Kombination des Penems (3) mit dem Cephalosporin (2) |
|---|---|
| E. coli 2136E | 0,5 |
| E. coli 2137E | 0,75* |
| E. coli 2138E | 0,5 |
| Citrobacter freundii 8090 | 0,281 |

* Der FIC-Wert von 0,75 spricht für eine überadditive Wirkung.

Beispiel 5

Die Kombination des Kaliumsalzes der Penem-Verbindung 5R,6S-6-(1R-Hydroxy-ethyl)-3-(4-formamidophenyl)-7-oxo-4-thia-1-azabicyclo[3.2.0]hept-2-en-2-carbonsäure (4) mit der Verbindung (2) wurde bei 3 Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte 0,5 für eine synergistische Aktivität charakteristisch, FIC-Werte von 1 zeigen eine additive Interaktion.

Tabelle 5

| Stamm | FIC-Werte einer Kombination des Penems (4) mit dem Cephalosporin (2) |
|---|---|
| E. coli 2136E | 0,5 |
| E. coli 2137E | 0,375 |
| E. coli 2138E | 0,5 |
| Citrobacter freundii 8090 | 0,091 |

Beispiel 6

Die Kombination des Natriumsalzes der Penem-Verbindung 5R,6S-3-(3-Carbamoyl-phenyl)-6-(1R-hydroxyethyl)-7-oxo-4-thia-1-azabicyclo[3.2.0]hept-2-en-2-carbonsäure (5) mit der Verbindung (2) wurde bei 3 Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte 0,5 für eine synergistische Aktivität charakteristisch, FIC-Werte von 1 zeigen eine additive Interaktion.

Tabelle 6

| Stamm | FIC-Werte einer Kombination des Penems (5) mit dem Cephalosporin (2) |
|---|---|
| E. coli 2136E | 0,375 |
| E. coli 2137E | 0,5 |
| E. coli 2138E | 0,375 |
| Citrobacter freundii 8090 | 0,125 |

Beispiel 7

Cephalosporine, wie oben als Beispiel angegeben, haben gegen grampositive Keime wie Staphylokokken und gegen gramnegative anaerobe Keime meist eine geringere Aktivität als gegen gramnegative

aerobe Krankheitserreger (siehe auch Seibert et al., Infection, Volume 11 (1983) 5, 275-279).

Peneme haben hingegen in der Regel eine bessere antibakterielle Aktivität gegen grampositive Kokken und Anaerobier als gegen gramnegative Keime (siehe auch Bauernfeind, J. Antimicrob. Chemother 15, 111, 1985).

Die Tabelle 7 zeigt für das Beispiel einer Kombination von einem Teil Penem und drei Teilen Cephalosporin eine Überlegenheit der antibakteriellen Aktivität der Kombination gegenüber den Einzelkomponenten. Das Kombinationspräparat gleicht die Schwäche im antibakteriellen Spektrum beider Präparate aus und hat somit ein breiteres antibakterielles Spektrum, das es für eine Therapie bakterieller Infektionen ohne vorherige Erregerisolierung besonders geeignet macht.

Tabelle 7

| Stamm | MHK in mg/l | | |
|---|---|---|---|
| | HRE 664 (1) | Cefotaxim (2) | (1) : (2) |
| | | | 1:3 |
| Strept. D 756 | 8 | 64 | 4 |
| Strept. D Eder | 8 | 64 | 4 |
| Strept. D 21777 | 8 | 64 | 4 |
| Strept. D 26777 | 8 | 64 | 4 |
| Citrob. 2901 | 8 | 64 | 4 |
| Citrob. 82 Cull. | 1 | 2 | 0,25 |
| Ent. cl. M 447 | 16 | 64 | 8 |
| Ent. cl. 2240 Cull. | 2 | 0,062 | 0,015 |
| E. coli 2139E | 1 | 0,031 | 0,015 |
| Kl. pneu. 1976E | 1 | 0,062 | 0,031 |
| B. vulgatus 1446 | 0,125 | 64 | 0,125 |
| B. distasonis 1366 | 0,25 | 0,125 | 0,125 |

Beispiel 8

Die Kombination von Natrium-5R,6S-6-(1R-hydroxyethyl)-3-(4-carbamoyl-phenoxy)-7-oxo-4-thia-1-azabicyclo[3.2.0]hept-2-en-2-carboxylat (1) mit Cefotaxim wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,312 |
| E. coli 2137E | 0,312 |
| E. coli 2138E | 0,249 |
| Citrobacter freundii 8090 | 0,094 |

Beispiel 9

Die Kombination von Natrium-5R,6S-6-(1R-hydroxyethyl)-3-(4-methyl-sulfinylphenyl)-7-oxo-4-thia-1-azabicyclo[3.2.1]hept-2-en-2-carboxylat (6) mit Cefotaxim (2) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.379 |
| E. coli 2137E | 0.5 |
| E. coli 2138E | 0.282 |
| Citrobacter freundii 8090 | 0.5 |

Beispiel 10

Die Kombination von Natrium-5R,6S-3-(4-methylsulfinylphenoxy)-6-(1R-hydroxyethyl)-7-oxo-4-thia-1-azabicyclo[3.2.0]hept-2-en-2-carboxylat (7) mit Cefotaxim (2) wurde bei drei Stämmen von E.coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.379 |
| E. coli 2137E | 0.320 |
| E. coli 2138E | 0.374 |
| Citrobacter freundii 8090 | 0.047 |

Beispiel 11

Die Kombination von Natrium-5R,6S-6-(1R-hydroxyethyl)-3-(3-sulfamoyl-propylthio)-7-oxo-4-thia-1-azabicyclo[3.2.0]hept-2-en-2-carboxylat (8) mit Cefotaxim (2) wurde bei drei Stämmen von E. coli und eines Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,379 |
| E. coli 2137E | 0,379 |
| E. coli 2138E | 0,379 |
| Citrobacter freundii 8090 | 0,312 |

Beispiel 12

Die Kombination von Kalium-5R,6S-6-(1R-hydroxyethyl)-3-(3-methylsulfinylphenyl)-7-oxo-4-thia-1-azabicyclo[3.2.0]hept-2-en-2-carboxylat (9) mit Cefotaxim (2) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.383 |
| E. coli 2137E | 0.383 |
| E. coli 2138E | 0.185 |
| Citrobacter freundii 8090 | 0.0935 |

Beispiel 13

Die Kombination von Natrium-5R,6S-3-(4-cyanophenoxy)-6-(1R-hydroxyethyl)-7-oxo-4-thia-1-azabicyclo-[3.2.0]hept-2-en-2-carboxylat (10) mit Cefotaxim (2) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,5 |
| E. coli 2137E | 0,25 |
| E. coli 2138E | 0,25 |
| Citrobacter freundii 8090 | 0,75 |

Beispiel 14

Die Kombination des Natriumsalzes von 5R,6S-6-(1R-Hydroxyethyl)-3-(4-carbamoyl-phenoxy)-7-oxo-4-thia-1-azabicyclo[3.2.0]hept-2-en-carbonsäure (1) mit Cefodizim (11) wurde bei drei Stämmen von E.coli und einem Stamm von Citrobacter freundii getestet. Als Maß für die synergistische Aktivität wurde die "fractional inhibitory concentration" (FIC-Index bestimmt nach Berenbaum, J Infect. Dis 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E.coli 2136E | 0,49 |
| E.coli 2137E | 0,5 |
| E.coli 2138E | 0.37 |
| Citrobacter freundii | 0,186 |

Beispiel 15

Die Kombination von Natrium-5R,6S-6-(1R-hydroxyethyl)-3-(4-methylsulfinylphenyl)-7-oxo-4-thia-1-azabicyclo[3.2.0]hept-2-en-2-carboxylat (6) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.155 |
| E. coli 2137E | 0.498 |
| E. coli 2138E | 0.374 |
| Citrobacter freundii 8090 | 0.375 |

Beispiel 16

Die Kombination von Natrium-5R,6S-3-(4-methylsulfinylphenoxy)-6-(1R-hydroxyethyl)-7-oxo-4-thia-1-azabicyclo[3.2.0]hept-2-en-2-carboxylat (7) mit Cefodizim (11) wurde bei drei Stämmen E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E coli 2136E | 0.5 |
| E. coli 2137E | 0.5 |
| E. coli 2138E | 0.249 |
| Citrobacter freundii 8090 | 0.140 |

Beispiel 17

Die Kombination von Kalium-5R,6S-6-(1R-hydroxyethyl)-3-(3-methylsulfinylphenyl)-7-oxo-4-thia-1-azabicyclo[3.2.0]hept-2-en-2-carboxylat (9) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.125 |
| E. coli 2137E | 0.187 |
| E. coli 2138E | 0.375 |
| Citrobacter freundii 8090 | 0.093 |

Beispiel 18

Die Kombination von Natrium-5R,6S-3-(4-cyanophenoxy)-6-(1R-hydroxy ethyl)-7-oxo-4-thia-1-azabicyclo-[3.2.0]hept-2-en-2-carboxylat (10) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,75 |
| E. coli 2137E | 0,373 |
| E. coli 2138E | 0,498 |
| Citrobacter freundii 8090 | 0,062 |

Beispiel 19

Die Kombination von Natrium-5R,6S-6-(1R-hydroxyethyl)-3-(3-sulfamoyl-propylthio)-7-oxo-4-thia-1-azabicyclo[3.2.0]hept-2-en-2-carboxylat (8) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,498 |
| E. coli 2137E | 0,498 |
| E. coli 2138E | 0,624 |
| Citrobacter freundii 8090 | 0,281 |

Beispiel 20

Die Kombination von Natrium-5R,6S-3-(3-carbamoylphenyl)-6-(1R-hydroxyethyl-7-oxo-4-thia-1-azabicyclo[3.2.0]hept-2-en-2-carboxylat (5) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,75 |
| E. coli 2137E | 0,496 |
| E. coli 2138E | 1 |
| Citrobacter freundii 8090 | 0,187 |

Beispiel 21

Die Kombination von Natrium-5R,6S-6-(1R-hydroxyethyl)-3-(2-carbamoyl-oxyethylthio)-7-oxo-4-thia-1-azabicyclo[3.2.0]hept-2-en-2-carboxylat (3) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.498 |
| E. coli 2137E | 0.498 |
| E. coli 2138E | 0.5 |
| Citrobacter freundii 8090 | 0.249 |

Beispiel 22

Die Kombination von Kalium-5R,6S-6-(1R-hydroxyethyl)-3-(4-formamidophenyl)-7-oxo-4-thia-1-azabicyclo[3.2.0]hept-2-en-2-carboxylat (4) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.75 |
| E. coli 2138E | 0.5 |
| Citrobacter freundii 8090 | 0.125 |

Beispiel 23

Die Kombination des Natriumsalzes von 5R,6S-6-(1R-hydroxyethyl)-3-(4-carbamoyl-phenoxy)-7-oxo-4-thia-1-azabicyclo[3.2.0]hept-2-en-2-carbonsäure (1) mit Cefpirom (12) wurde bei drei Stämmen von E.coli und einem Stamm von Citrobacter freundii getestet. Als Maß für die synergistische Aktivität wurde die "fractional inhibitory concentration" (FIC-Index bestimmt nach Berenbaum, J Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E.coli 2136E | 0,5 |
| E.coli 2137E | 0,66 |
| E.coli 2138E | 0,315 |
| Citobacter freundii | 0,5 |

Beispiel 24

Die Kombination von Natrium-5R,6S-6-(1R-hydroxyethyl)-3-(4-methylsulfinylphenyl)-7-oxo-4-thia-1-azabicyclo[3.2.0]hept-2-en-2-carboxylat (6) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0.5 für eine synergistische Aktivität charakteristisch, FIC-Werte von 1 zeigen eine additive Interaktion.

| Stamm | FIC-Index |
|---|---|
| E` coli 2136E | 0.374 |
| E. coli 2137E | 0.249 |
| E. coli 2138E | 0.187 |
| Citrobacter freundii 8090 | 0.126 |

Beispiel 25

Die Kombination von Natrium-5R,6S-3-(4-methylsulfinylphenoxy)-6-(1R-hydroxyethyl)-7-oxo-4-thia-1-azabicyclo[3.2.0]hept-2-en-2-carboxylat (7) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 Zeigen eine additive Interaktion.

| Stamm | FIC-Index |
|---|---|
| E. coli 2136E | 0.629 |
| E. coli 2137E | 0.5 |
| E. coli 2138E | 0.5 |
| Citrobacter freundii 8090 | 0.126 |

Beispiel 26

Die Kombination von Kalium-5R,6S-6-(1R-hydroxyethyl)-3-(3-methylsulfinylphenyl)-7-oxo-4-thia-1-azabicyclo[3.2.0]hept-2-en-2-carboxylat (9) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.125 |
| E. coli 2137E | 0.5 |
| E. coli 2138E | 0.186 |
| Citrobacter freundii 8090 | 0.258 |

Beispiel 27

Die Kombination von Natrium-5R,6S-3-(4-cyanophenoxy)-6-(1R-hydroxyethyl)-7-oxo-4-thia-1-azabicyclo-[3.2.0]hept-2-en-2-carboxylat (10) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,5 |
| E. coli 2137E | 0,5 |
| E. coli 2138E | 0,5 |
| Citrobacter freundii 8090 | 0,062 |

Beispiel 28

Die Kombination von Natrium-5R,6S-6-(1R-hydroxyethyl)-3-(3-sulfamoyl-propylthio)-7-oxo-4-thia-1-azabicyclo[3.2.0]hept-2-en-2-carboxylat (8) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte Von 1 zeigen eine additive Interaktion.

| Stamm | FIC-Index |
|---|---|
| E. coli 2136E | 0,75 |
| E. coli 2137E | 0,62 |
| E. coli 2138E | 0,5 |
| Citrobacter freundii 8090 | 0,5 |

Beispiel 29

Die Kombination von Natrium-5R,6S-3-(3-carbamoyl-phenyl)-6-(1R-hydroxyethyl)-7-oxo-4-thia-1-azabicyclo[3.2.0]hept-2-en-2-carboxylat (5) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,5 |
| E. coli 2137E | 0,75 |
| E. coli 2138E | 0,372 |
| Citrobacter freundii 8090 | 0,390 |

Beispiel 30

Die Kombination von Natrium-5R,6S-6-(1R-hydroxyethyl)-3-(2-carbamoyloxyethyl)-7-oxo-4-thia-1-azabicyclo[3.2.0]hept-2-en-2 carboxylat (3) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.366 |
| E. coli 2137E | 0.496 |
| E. coli 2138E | 0.372 |
| Citrobacter freundii 8090 | 0.366 |

Beispiel 31

Die Kombination von Kalium-5R,6S-6-(1R-hydroxyethyl)-3-(4-formamidophenyl)-7-oxo-4-thia-1-azabicyclo[3.2.0]hept-2-en-2-carboxylat (4) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.5 |
| E. coli 2138E | 0.312 |
| Citrobacter freundii 8090 | 0.125 |

Beispiel 32

Die Kombination des Natriumsalzes von 5R,6S-6-(1R-Hydroxyethyl)-3-(4-carbamoyl-phenoxy)-7-oxo-4-thia-1-azabicyclo[3.2.0]hept-2-en-2-carbonsäure (1) mit Ceftriaxon (13) wurde bei drei Stämmen von E.coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,5 |
| E. coli 2137E | 0,379 |
| E. coli 2138E | 0,75 |
| Citrobacter freundii 8090 | 0,075 |

Beispiel 33

Die Kombination von Natrium-5R,6S-6-(1R-hydroxyethyl)-3-(4-methylsulfinylphenyl)-7-oxo-4-thia-1-azabicyclo[3.2.0]hept-2-en-2-carboxylat (6) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.498 |
| E. coli 2138E | 0.374 |
| Citrobacter freundii 8090 | 0.187 |

Beispiel 34

Die Kombination von Natrium-5R,6S-3-(4-methylsulfinylphenoxy)-6-(1R-hydroxyethyl)-7-oxo-4-thia-1-azabicyclo/3.2.0/hept-2-en-2-carboxylat (7) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0.5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.5 |
| E. coli 2138E | 0.75 |
| Citrobacter freundii 8090 | 0.091 |

Beispiel 35

Die Kombination von Kalium-5R,6S-6-(1R-hydroxyethyl)-3-(3-methylsulfinylphenyl)-7-oxo-4-thia-1-azabicyclo/3.2.0/hept-2-en-2-carboxylat (9) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.125 |
| E. coli 2137E | 0.75 |
| E. coli 2138E | 0.077 |
| Citrobacter freundii 8090 | 0.034 |

Beispiel 36

Die Kombination von Natrium-5R,6S-3-(4-cyanophenoxy)-6-(1R-hydroxyethyl)-7-oxo-4-thia-1-azabicyclo-[3.2.0]hept-2-en-2-carboxylat (10) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,5 |
| E. coli 2137E | 0,5 |
| E. coli 2138E | 0,5 |
| Citrobacter freundii 8090 | 0,182 |

Beispiel 37

Die Kombination von Natrium-5R,6S-6-(1R-hydroxyethyl)-3-(3-sulfamoyl-propylthio)-7-oxo-4-thia-1-azabicyclo[3.2.0]hept-2-en-2-carboxylat (8) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,5 |
| E. coli 2137E | 0,5 |
| E. coli 2138E | 0,5 |
| Citrobacter freundii 8090 | 0,186 |

Beispiel 38

Die Kombination von Natrium-5R,6S-3-(3-carbamoyl-phenyl)-6-(1R-hydroxyethyl)-7-oxo-4-thia-1-azabicyclo[3.2.0]hept-2-en-2-carboxylat (5) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0,318 |
| E. coli 2137E | 0,31 |
| E. coli 2138E | 0,75 |
| Citrobacter freundii 8090 | 0,06 |

Beispiel 39

Die Kombination von Natrium-5R,6S-6-(1R-hydroxyethyl)-3-(2-carbamoyloxyethylthio)-7-oxo-4-thia-1-azabicyclo[3.2.0]hept-2-en-2-carboxylat (3) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.75 |
| E. coli 2137E | 0.75 |
| E. coli 2138E | 0.629 |
| Citrobacter freundii 8090 | 0.0625 |

Beispiel 40

Die Kombination von Kalium-5R,6S-6-(1R-hydroxyethyl)-3-(4-formamidophenyl)-7-oxo-4-thia-1-azabicyclo[3.2.0]hept-2-en-2-carboxylat (4) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.125 |
| E. coli 2138E | 0.5 |
| Citrobacter freundii 8090 | 0.372 |

Beispiel 41

Die Kombination von Natrium-5R,6S-3-(4-formamido-phenoxy)-6-(1R-hydroxyethyl)-7-oxo-4-thia-1-azabicycio[3.2.0]hept-2-en-2-carboxylat (16) mit Cefotaxim (2) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.383 |
| E. coli 2137E | 0.5 |
| E. coli 2138E | 0.374 |
| Citrobacter freundii 8090 | 0.122 |

Beispiel 42

Die Kombination von Natrium-5R,6S-3-(4-formamido-phenoxy)-6-(1R-hydroxyethyl)-7-oxo-4-thia-1-azabicyclo[3.2.0]hept-2-en-2-carboxylat (16) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.5 |
| E. coli 2138E | 0.125 |
| Citrobacter freundii 8090 | 0.047 |

Beispiel 43

Die Kombination von Natrium-5R,6S-3-(4-formamido-phenoxy)-6-(1R-hydroxyethyl)-7-oxo-4-thia-1-azabicyclo[3.2.0]hept-2-en-2-carboxylat (16) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. bis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.5 |
| E. coli 2138E | 0.5 |
| Citrobacter freundii 8090 | 0.273 |

Beispiel 44

Die Kombination von Natrium-5R,6S-3-(4-formimido-Phenoxy)-6-(1R-hydroxyethyl)-7-oxo-4-thia-1-azabicyclo[3.2.0]hept-2-en-2-carboxylat (16) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.5 |
| E. coli 2138E | 0.36 |
| Citrobacter freundii 8090 | 0.122 |

Beispiel 45

Die Kombination von Natrium-5R,6S-3-(carbamoyloxymethyl)-6-(1R-hydroxyethyl)-7-oxo-4-thia-1-azabicyclo[3.2.0]hept-2-en-2-carboxylat (17) mit Cefotaxim (2) wurde bei drei Stämmen von E. coli und einen Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.5 |
| E. coli 2137E | 0.37 |
| E. coli 2138E | 0.5 |
| Citrobacter freundii 8090 | 0.122 |

Beispiel 46

Die Kombination von Natrium-5R,6S-3-(carbamoyloxymethyl)-6-(1R-hydroxyethyl)-7-oxo-4-thia-1-azabicyclo[3.2.0]hept-2-en-2 carboxylat (17) mit Cefodizim (11) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E coli 2136E | 0.5 |
| E. coli 2137E | 0.5 |
| E. coli 2138E | 0.75 |
| Citrobacter freundii 8090 | 0.125 |

Beispiel 47

Die Kombination von Natrium-5R,6S-3-(carbamoyloxymethyl)-6-(1R-hydroxyethyl)-7-oxo-4-thia-1-azabicyclo[3.2.0]hept-2-en-2-carboxylat (17) mit Cefpirom (12) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.37 |
| E. coli 2137E | 0.5 |
| E. coli 2138E | 0.5 |
| Citrobacter freundii 8090 | 0.062 |

Beispiel 48

Die Kombination von Natrium-5R,6S-3-(carbamoyloxymethyl)-6-(1R-hydroxyethyl)-7-oxo-4-thia-1-azabicyclo[3.2.0]hept-2-en-2-carboxylat (17) mit Ceftriaxon (13) wurde bei drei Stämmen von E. coli und einem Stamm von Citrobacter freundii getestet. Als Maß für die synergistische Aktivität wurde die "fractional inhibitory concentration" (FIC-Index) bestimmt nach Berenbaum, J. Infect. Dis. 137 (1978), 122-136. Danach sind FIC-Werte ≦ 0,5 für eine synergistische Aktivität charakteristisch, FIC-Werte von 1 zeigen eine additive Interaktion.

| Stamm | FIC-Index |
|---|---|
| E. coli 2136E | 0.375 |
| E. coli 2137E | 0.125 |
| E. coli 2138E | 0.5 |
| Citrobacter freundii 8090 | 0.093 |

Beispiel 49

Herstellung einer parenteralen Zubereitung

1,5 g Cefotaxim (2) und 0,5gHRE 664 (1) werden in 10 ml Wasser ad injec. gelöst und anschließend appliziert.

In Abhängigkeit von den Löslichkeitsverhältnissen gelten ähnliche Mengenverhältnisse auch für die anderen, vorstehend beschriebenen Ausführungsbeispiele.

Verbindungen der Ausführungsbeispiele

**Verbindungsnummer**

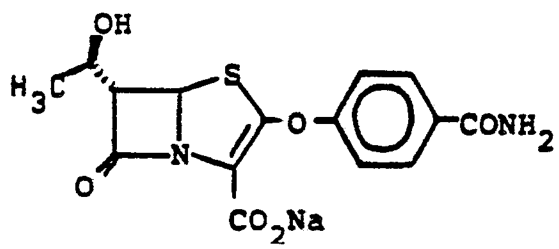

**(1)**
**HRE 664**

5R,6S-6-(1R-Hydroxyethyl)-3-(4-carbamoyl-phenoxy)-7-oxo-4-thia-1-azabicyclo[3.2.0]hept-2-en-2-carbonsäure (Natriumsalz)

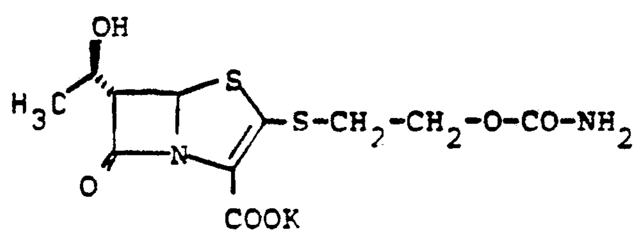

**(3)**

5R,6S-3-(2-Carbamoyloxyethylthio)-6-(1R-hydroxyethyl)-7-oxo-4-thia-1-azabicyclo[3.2.0]hept-2-en-2-carbonsäure (Kaliumsalz)

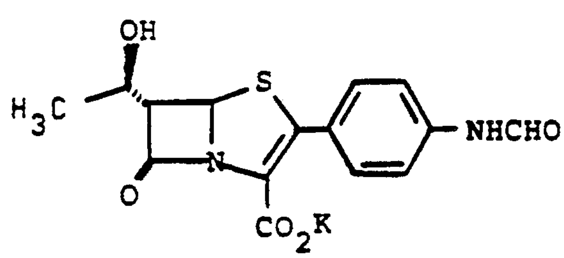

**(4)**

5R,6S-6-(1R-Hydroxyethyl)-3-(4-formamidophenyl)-7-oxo-4-thia-1-azabicyclo[3.2.0]hept-2-en-2-carbonsäure (Kaliumsalz)

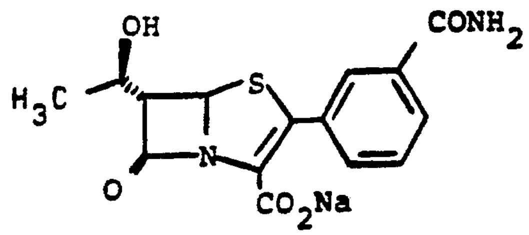

(5)

5R,6S-3-(3-Carbamoyl-phenyl)-6-(1R-hydroxyethyl)-7-oxo-4-thia-1-azabicyclo[3.2.0]hept-2-en-2-carbonsäure (Natriumsalz)

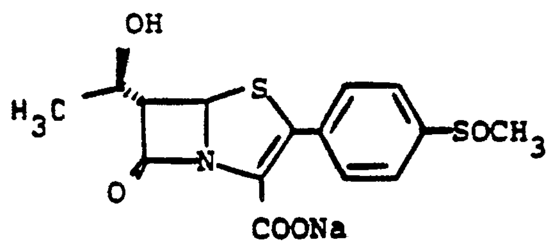

(6)

5R,6S-6-(1R-Hydroxyethyl)-3-(4-methylsulfinyl-phenyl)-7-oxo-4-thia-1-azabicyclo[3.2.0]hept-2-en-2-carbonsäure (Natriumsalz)

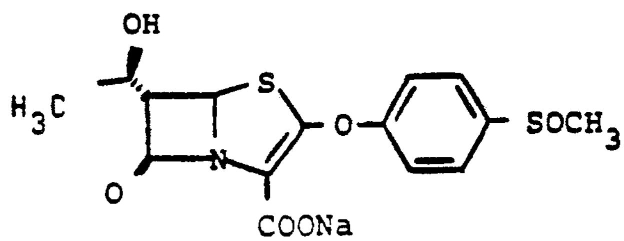

(7)

5R,6S-3-(4-Methylsulfinyl-phenoxy)-6-(1R-hydroxyethyl)-7-oxo-4-thia-1-azabicyclo[3.2.0]hept-2-en-2-carbonsäure (Natriumsalz)

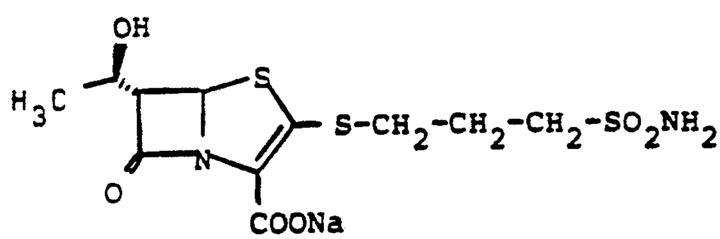

(8)

5R,6S-6-(1R-Hydroxyethyl)-3-(3-sulfamoyl-propylthio)-7-oxo-4-thia-1-azabicyclo[3.2.0]hept-2-en-2-carbonsäure (Natriumsalz)

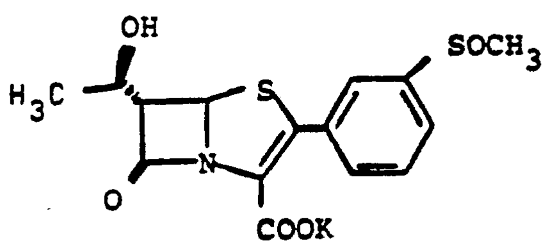

(9)

5R,6S-6-(1R-Hydroxyethyl)-3-(3-methylsulfinyl-phenyl)-7-oxo-4-thia-1-azabicyclo[3.2.0]hept-2-en-2-carbonsäure (Kaliumsalz)

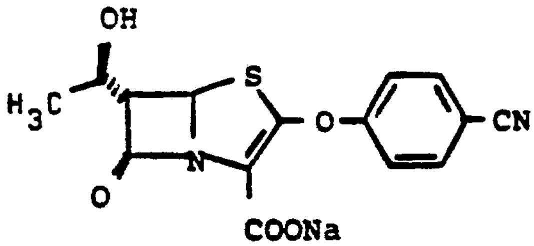

(10)

5R,6S-3-(4-Cyanophenoxy)-6-(1R-hydroxyethyl)-7-oxo-4-thia-1-azabicyclo[3.2.0]hept-2-en-2-carbonsäure (Natriumsalz)

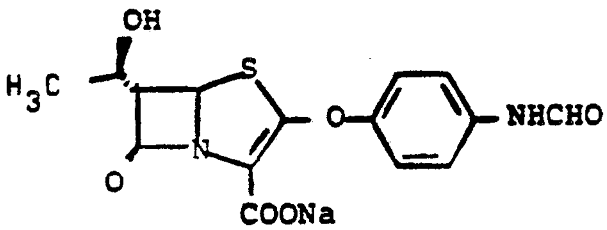

(16)

5R,6S-3-(4-Formamido-phenoxy)-6-(1R-hydroxyethyl)-7-oxo-4-thia-1-azabicyclo[3.2.0]hept-2-en-2-carbonsäure (Natriumsalz)

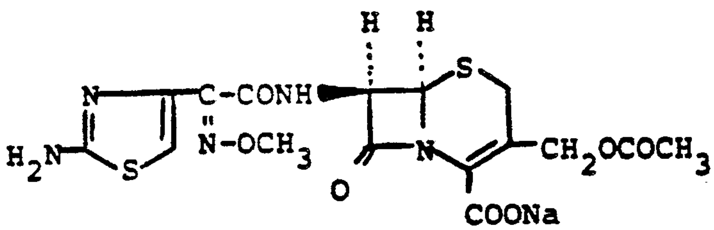

(2)

Cefotaxim (Natriumsalz)

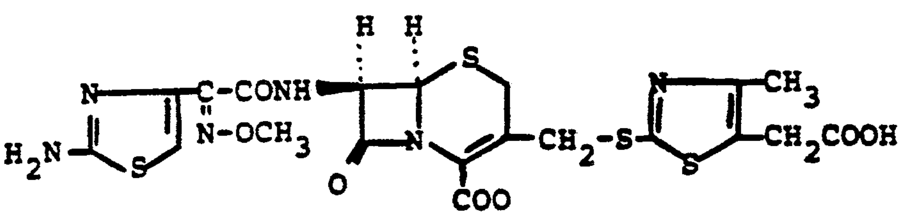

(11)

Cefodizim (Natriumsalz)

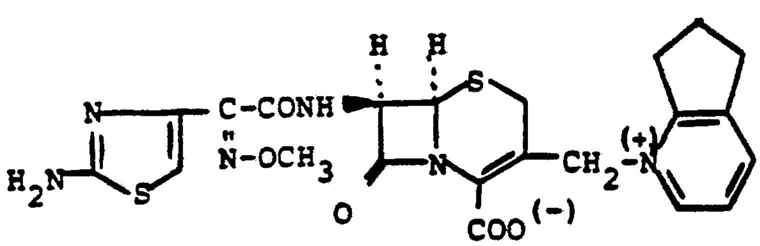

(12)

Cefpirom

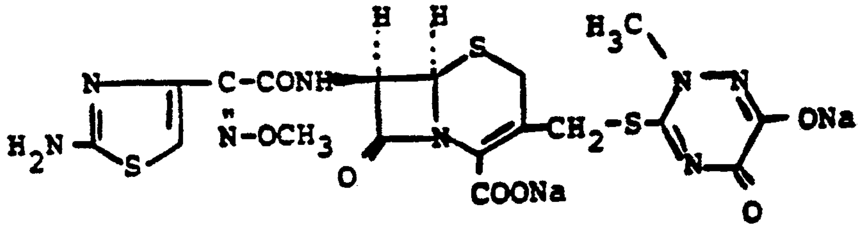

(13)

Ceftriaxon (Dinatriumsalz)

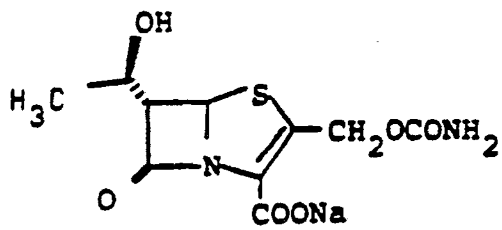

(17)

5R,6S-3-(Carbamoyloxmethyl)-6-(1R-hydroxyethyl)-7-oxo-4-thia-1-azabicyclo[3.2.0]hept-2-en-2-carbonsäure (Natriumsalz)

**Patentansprüche**

1. Pharmazeutsiche Zubereitung mit synergistischer antibakterieller Wirksamkeit, enthaltend ein Cephalosporin und ein Penemantibiotikum, dadurch gekennzeichnet, daß
   (a) ein Cephalosporinderivat der allgemeinen Formel I

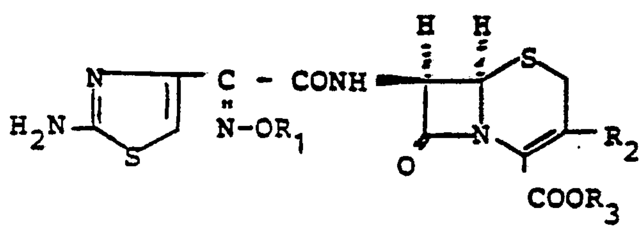

(I)

worin

$R_1$ für Wasserstoff,
$C_1$-$C_4$-Alkyl oder Carboxy-$C_1$-$C_4$-alkyl, und die Gruppe =N-$OR_1$ in syn-Position steht,

$R_2$ die Bedeutung hat von Wasserstoff, Methyl, Methoxy, Vinyl, Acetoxymethyl, Carbamoyloxymethyl, von -$CH_2$S-X,
mit X =

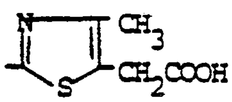 , 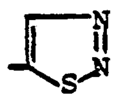 , 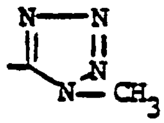 , 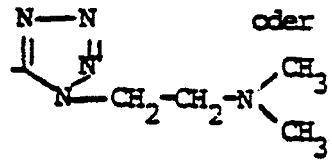 oder 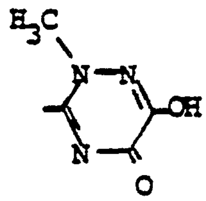

von 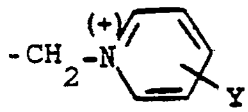 mit Y = Wasserstoff, $C_1$-$C_4$-Alkylthio, $C_1$-$C_4$-Alkoxy oder $C_3$-$C_5$-Cycloalkyl,

von 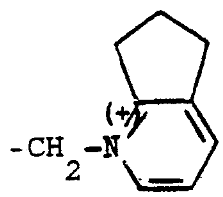 oder 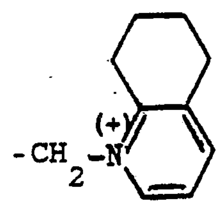 ,

wobei die ankondensierten Ringe auch in 3,4-Stellung stehen und auch durch Sauerstoff unterbrochen sein können,
von

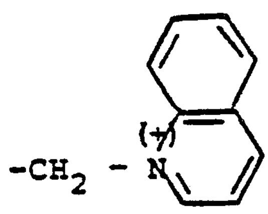 oder 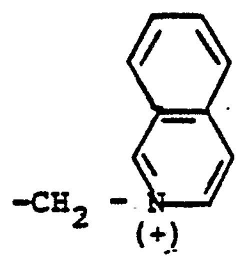

von Thienopyridinio-methyl, Furopyridinio-methyl oder von 5-Methyl-tetrazol-2-yl-methyl und
$R_3$ für Wasserstoff, ein physiologisch verträgliches Kation, einen physiologisch verträglichen Esterrest oder - falls in $R_2$ die Struktur

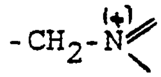

auftritt - für eine negative Ladung steht, und
(b) ein Penem-Antibiotikum der allgemeinen Formel II

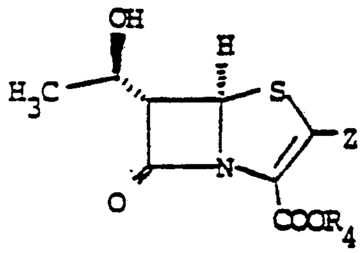

(II)

worin

Z steht für 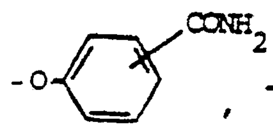 , 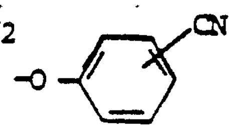 , 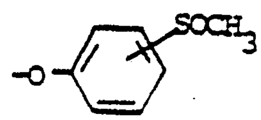 ,

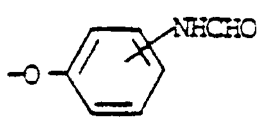 , 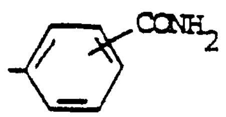 , 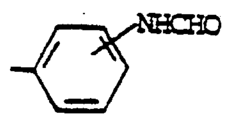 , 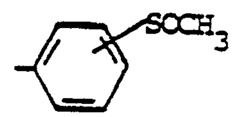 ,

$-CH_2-O-CONH_2$, $-S-(CH_2)_2-O-CONH_2$ oder $-S-(CH_2)_3-SO_2NH_2$ und

$R_4$ für Wasserstoff, ein physiologisch verträgliches Kation oder einen physiologisch verträglichen Esterrest,

vorliegen.

2. Verfahren zur Herstellung einer Zubereitung gemäß Anspruch 1, dadurch gekennzeichnet, daß man
   a) ein Cephalosporinderivat der allgemeinen Formel I gemäß Anspruch 1 oder dessen physiologisch verträgliche Salze oder Ester und
   b) ein Penem-Antibiotikum der allgemeinen Formel II gemäß Anspruch 1 oder dessen physiologisch verträgliche Salze oder Ester

   zusammen mit einem physiologisch verträglichen Träger und gegebenenfalls weiteren Hilfs- oder Zusatzstoffen in eine geeignete Darreichungsform bringt.

3. Pharmazeutische Zubereitung gemäß Anspruch 1, dadurch gekennzeichnet, daß sie nebeneinander in unvermischter Form
   a) ein Cephalosporinderivat der allgemeinen Formel I gemäß Anspruch 1 oder dessen physiologisch verträgliche Salze oder Ester und
   b) ein Penem-Antibiotikum der allgemeinen Formel II gemäß Anspruch 1 oder dessen physiologisch verträgliche Salze oder Ester enthält und

   ein Kombinationspräparat zur gleichzeitigen,getrennten oder zeitlich abgestuften Anwendung bei der Behandlung bakterieller Infektionen darstellt.

**Claims**

1. A pharmaceutical formulation having a synergistic antibacterial activity, containing a cephalosporin and a penem antibiotic, wherein
   a) a cephalosporin derivative of the formula I

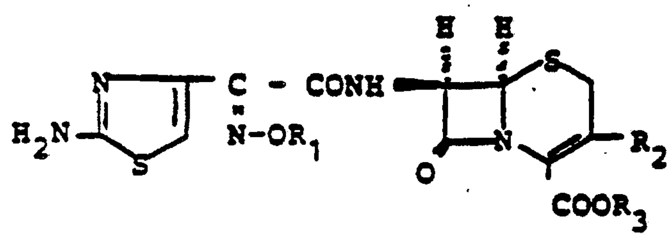 (I)

in which

- $R_1$ is hydrogen, $C_1$-$C_4$-alkyl or carboxy-$C_1$-$C_4$-alkyl and the $=N-OR_1$ group is in the synposition,
- $R_2$ has the meaning of hydrogen, methyl, methoxy, vinyl, acetoxymethyl or carbamoyloxymethyl, of $-CH_2S-X$, with X =

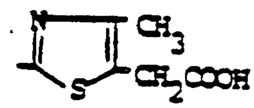 , 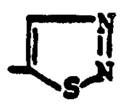 , 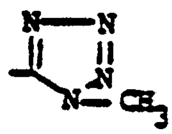 , 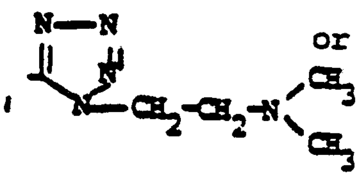 or

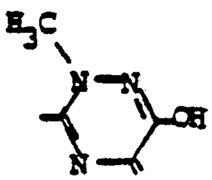

of 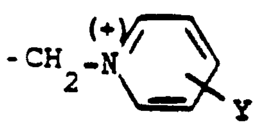 with Y = hydrogen, $C_1$-$C_4$-alkylthio, $C_1$-$C_4$-alkoxy or $C_3$-$C_5$-cycloalkyl,

of 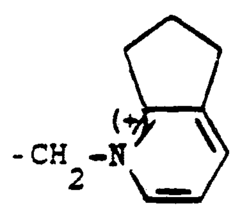 or 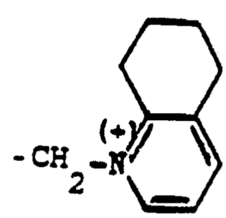

it being possible for the fused rings also to be in the 3,4-position and also to be interrupted by oxygen, of

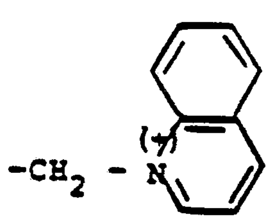 or 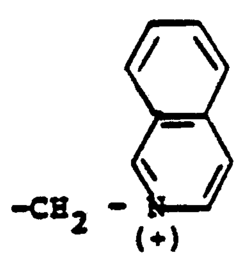

of thienopyridinio-methyl, furopyridinio-methyl or of 5-methyl-tetrazol-2-yl-methyl

and

$R_3$ is hydrogen, a physiologically acceptable cation, a physiologically acceptable ester radical or -if the structure

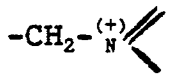

appears in $R_2$ - is a negative charge, and

b) a penem antibiotic of the formula II

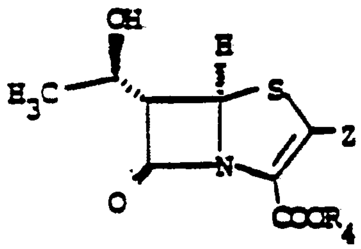

(II)

in which

Z is 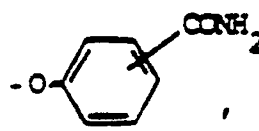 , 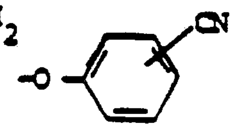 , 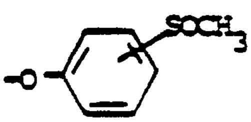 ,

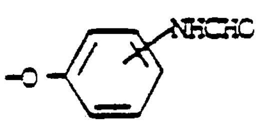 , 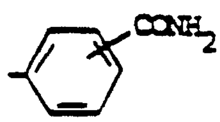 , 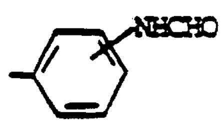 , 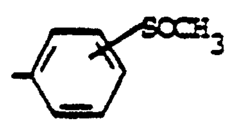 ,

$-CH_2-O-CONH_2$, $-S-(CH_2)_2-O-CONH_2$ or $-S-(CH_2)_3-SO_2NH_2$

and

$R_4$ is hydrogen, a physiologically acceptable cation or a physiologically acceptable ester radical,

are present.

**2.** A process for preparing a formulation as claimed in claim 1, which comprises bringing

a) a cephalosporin derivative of the formula I as claimed in claim 1 or a physiologically acceptable salt or ester thereof and

b) a penem antibiotic of the formula II as claimed in claim 1 or a physiologically acceptable salt or ester thereof

together with a physiologically acceptable excipient and, if appropriate, further auxiliaries or additives into a suitable administration form.

**3.** A pharmaceutical formulation as claimed in claim 1 containing side by side in an unmixed form

a) a cephalosporin derivative of the formula I as claimed in claim 1 or a physiologically acceptable salt or ester thereof and

b) a penem antibiotic of the formula II as claimed in claim 1 or a physiologically acceptable salt or ester thereof

as a combination product for simultaneous or separate application or for application spaced out in time in the treatment of bacterial infections.

**Revendications**

1. Compositions pharmaceutique à activité antibactérienne synergique, contenant une céphalosporine et un antibiotique de type pénème, caractérisée en ce que sont présents

(a) un dérivé de céphalosporine de formule générale I

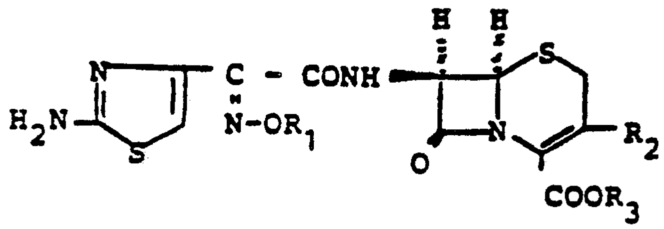

(I)

dans laquelle

$R_1$ représente un atome d'hydrogène ou un groupe alkyle en $C_1$-$C_4$ ou carboxyalkyle($C_1$-$C_4$), et le groupe =N-$OR_1$ est en position syn,

$R_2$ représente un atome d'hydrogène ou un groupe méthyle, méthoxy, vinyle, acétoxyméthyle, carbamoyloxyméthyle, un groupe -$CH_2$ S-X, avec X=

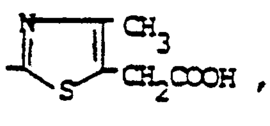 , 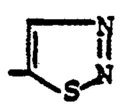 , 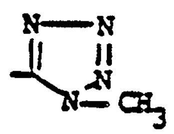 , 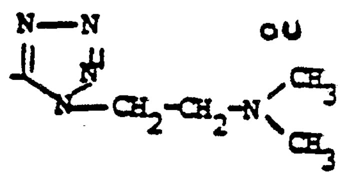 ou 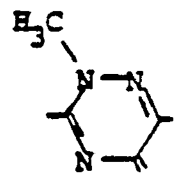

un groupe 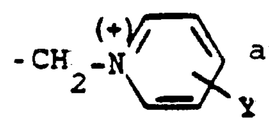 avec Y = un atome d'hydrogène, un groupe alkyl($C_1$-$C_4$)-thio, alcoxy en $C_1$-$C_4$ ou cycloalkyle en $C_3$-$C_5$

un groupe 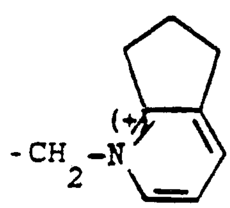 ou 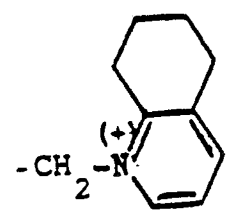

les cycles condensés pouvant également se trouver en position 3,4 et être également interrompus par un atome d'hydrogène,

un groupe 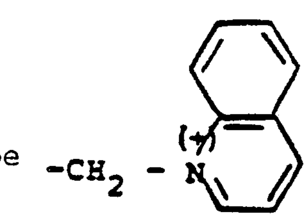 ou 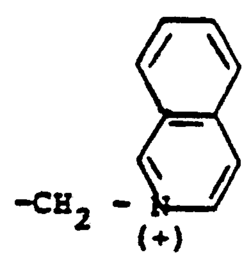

le groupe thiénopyridiniométhyle, furopyridiniométhyle ou 5-méthyl-tétrazol-2-yl-méthyle, et

$R_3$ représente un atome d'hydrogène, un cation physiologiquement acceptable, un reste ester physiologiquement acceptable ou - au cas où la structure

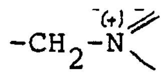

apparaît dans $R_2$ - une charge négative, et

(b) un antibiotique de type pénème, de formule générale II

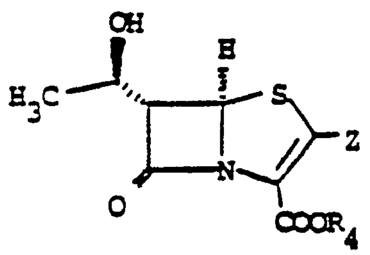

(II)

dans laquelle

Z représente

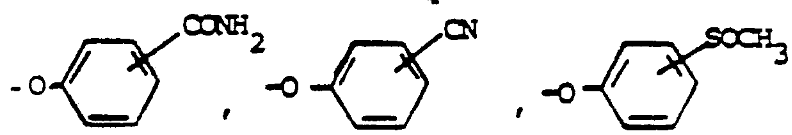

, , ,

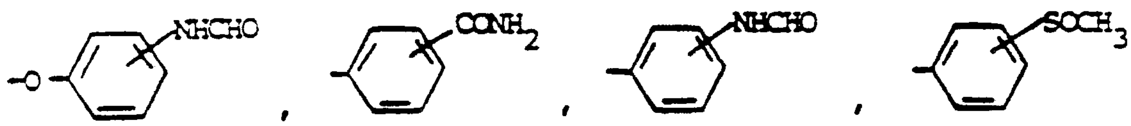

, , , ,

$-CH_2-O-CONH_2$, $-S-(CH_2)_2-O-CONH_2$ ou $-S-(CH_2)_3-SO_2NH_2$

et

$R_4$ représente un atome d'hydrogène, un cation physiologiquement acceptable ou un reste ester physiologiquement acceptable.

**2.** Procédé pour la préparation d'une composition selon la revendication 1, caractérisé en ce que l'on met sous une forme pharmaceutique appropriée

a) un dérivé de céphalosporine, de formule générale I, selon la revendication 1, ou ses sels ou esters physiologiquement acceptables, et

b) un antibiotique de type pénème, de formule générale II, selon la revendication 1, ou ses sels ou esters physiologiquement acceptables

conjointement avec un(des) véhicule(s) physiologiquement acceptable(s) et éventuellement d'autres additifs ou adjuvants.

**3.** Composition pharmaceutique selon la revendication 1, caractérisée en ce qu'elle contient, l'un à côté de l'autre, sous forme non mélangée,

a) un dérivé de céphalosporine, de formule générale I, selon la revendication 1, ou ses sels ou esters physiologiquement acceptables, et

b) un antibiotique de type pénème, de formule générale II, selon la revendication 1, ou ses sels ou esters physiologiquement acceptables, et

représente une composition mixte pour l'administration simulanée, séparée ou échelonnée dans le temps, dans le traitement d'infections bactériennes.